# EUROPÄISCHE PATENTANMELDUNG

(11) **EP 1 240 909 A1**
(43) Veröffentlichungstag der Anmeldung: **18.09.2002**
(21) Anmeldenummer: 02004468.1
(22) Anmeldetag: 27.02.2002
(51) Int. Cl.: A61M 1/00, A61B 17/32

(54) **Instrument zum Absaugen von Körperfett**

(30) Priorität: 16.03.2001 DE 10112735
(71) Anmelder: Möller Feinmechanik GmbH & Co. KG, 36043 Fulda (DE)
(72) Erfinder: Schröter, Werner, 36137 Grossenlüder (DE)
(74) Vertreter: Hebing, Norbert

(57) **Zusammenfassung**

Es wird ein Instrument zum Absaugen von Körperfett beschrieben. Der Griff (1) besteht aus einem Hauptteil (2) sowie zwei Anschlussstücken (4, 5), die über gleichartig aufgebaute Verbindungen miteinander verbunden sind. Um das Instrument mit einem Vibrationsantriebssystem koppeln zu können, wird der Hauptteil (2) entfernt und die beiden Anschlussstücke (4, 5) zusammengesetzt, so dass ein Greifer des Vibrationsantriebssystems den so gebildeten Körper erfassen kann. Der Vorteil bei der Anordnung besteht darin, dass bei einer manuellen Handhabe ein vollständiger Griff für den Operateur vorliegt. Bei einer Maßnahme mit Vibration besitzt der Körper, der mit dem Vibrationsantriebssystem gekoppelt wird, nur ein geringes Gewicht, so dass keine hohen Antriebsleistungen für das Vibrationsantriebssystem notwendig sind.

## Beschreibung

Die Erfindung bezieht sich auf ein Instrument zum Absaugen von Körperfett mit einer hohlen Kanüle und einem einen Hauptteil und zwei Anschlussstücke aufweisenden hohlen Griff, wobei an das eine Anschlussstück eine Saugpumpe anschließbar und das andere Anschlussstück mit der Kanüle verbunden ist.

Ein derartiges Instrument ist in der US-PS 4,568,332 beschrieben. Die beiden Anschlussstück sind in dieser Ausführung einteilig mit dem Hauptteil des Griffes ausgeführt. Auch wenn in der Schrift nicht explizit darauf hingewiesen wird, werden derartige Instrumente aus einem Metall gefertigt, was es erlaubt, sie mit Dampf bei Drücken bis zu 2 bar und Temperaturen bis zu 125°C zu sterilisieren. Die Herstellung aus Metall bedeutet aber, dass das Instrument eine gewisse Schwere aufweist. Bis zu einem gewissen Grad ist dieses sogar erwünscht, da das Instrument dann gut in der Hand des die Absaugung vornehmenden Operateurs liegt. Für die folgende Weiterbildung des Instruments ist ein zu hohes Gewicht aber nachteilig: Um die Absaugleistung zu verbessern, wird nämlich vorgesehen, das Instrument mit einem Vibrationsantriebssystem zu verbinden, so dass die Kanüle ins Längsrichtung einen Hub von wenigen Millimetern mit einer Frequenz von etwa 7.000 Hüben pro Minute durchführt. Die Leistungsabgabe des Vibrationsantriebssystem muss allerdings um so größer sein, je schwerer das Instrument ist, da bei jedem Hub das schwere Instrument erneut beschleunigt werden muss Für eine vibrationsgestützte Anwendung sollte das Instrument daher möglichst leicht aufgebaut sein.

Die Erfindung beruht somit auf dem Problem, ein Instrument zu schaffen, das einerseits gut vom Operateur zu handhaben ist, andererseits aber auch zusammen mit einem Vibrationsantriebssystem genutzt werden kann und dass außerdem in Heißdampf sterilsiert werden kann.

Dazu sieht die Erfindung ein Instrument gemäß dem Oberbegriff des Anspruchs 1 vor, dass dadurch gekennzeichnet ist, dass die beiden Anschlussstücke des Griffes vom Hauptteil des Griffes lösbar und zu einem mit einem Vibrationsantriebssystem koppelbaren Körper zusammenbringbar sind.

Benutzt der Operateur das Instrument ohne ein Vibrationsantriebssystem anzuschließen, so besteht der Griff aus dem Hauptteil und den Anschlussstücken. Damit weist der Griff eine gewisse Schwere und Abmessung auf, so dass die Kanüle vom Operateur sicher geführt werden kann. Beim Einsatz eines Vibrationsantriebssystems werden die beiden Anschlussstücke vom Hauptteil gelöst und miteinander verbunden, so dass ein relativ leichter Körper entsteht, der mit dem Vibrationsantriebssystem gekoppelt werden kann. Die von dem Vibrationsantriebssystem zu bewegende Masse ist damit klein und die Leistungsabgabe kann gering gehalten werden. Außerdem ist die Rückwirkung auf das Vibrationsantriebssystem gering, so dass dieses ruhig in der Hand des Operateurs liegt.

Damit der Wechsel von einem vollständigen Griff auf einen mit dem Vibrationsantriebssystem koppelbaren Körper und umgekehrt rasch erfolgen kann, wird vorgeschlagen, dass die Anschlussstücke über gleichartig aufgebaute, jeweils aus zwei korrespondierenden Verbindungselementen bestehenden Verbindungen mit dem Hauptteil koppelbar sind. Dies erlaubt es, dieselben Verbindungselemente zum Verbinden der Anschlussstücke mit dem Hauptteil des Griffes und für die Verbindung der Anschlussstücke untereinander zu nutzen, was einen einfachen Aufbau des Instrumentes zur Folge hat.

Die Verbindungen müssen luftdicht sein, so dass die Saugwirkung nicht beeinträchtigt wird. Daher wird vorgeschlagen, dass es sich um Schraubverbindungen handelt, die jeweils aus einem Innen- und einem Außengewinde bestehen, wobei am Hauptteil des Griffes ein Innengewinde und ein Außengewinde ausgebildet ist. Damit besteht an dem einen Anschlussstück ein Außengewinde und an dem anderen ein Innengewinde, die zueinander passen.

Für eine einfache Handhabung haben sich Instrumente mit einem runden Querschnitt bewährt. Es wird daher vorgeschlagen, dass der Hauptteil des Griffes aus einem Rohr besteht und die Anschlussstücke im Anschluss an den Hauptteil aus Hohlzylindern, wobei die Außendurchmesser des Hohlzylinders und des Rohres gleich sind. Damit stellt sich ein glatter Übergang ein, was die Handhabbarkeit des Griffes deutlich erhöht.

Um den Halt des Operateurs zu verbessern, wurde schon vorgeschlagen, den Hauptteil des Griffes mit einer Riffelung zu versehen. Um die Griffigkeit noch weiter zu erhöhen wird zusätzlich vorgeschlagen, den Hohlzylinder des Anschlussstückes für den Kanülenanschluss mit einer geriffelten Mantelfläche zu versehen. Dies hat den Vorteil, dass ein Griffstück des Vibrationsantriebssystems sicher an den aus den beiden Anschlussstücken bestehenden Körpern gehalten werden kann.

Während einer Operation wird mit unterschiedlich dicken Kanülen gearbeitet, je nachdem in welchen Bereichen des Körpers die Absaugung erfolgt. Um die Kanülen leicht auswechseln zu können, ist das Anschlussstück für die Kanüle nicht lösbar mit dieser verbunden. Zum Austauschen der Kanüle wird das Anschlussstück mit der Kanüle entweder aus dem Hauptteil des Griffes oder aus dem Anschlussstück mit dem Sauganschluss herausgeschraubt.

Im Folgenden wird anhand eines Ausführungsbeispiels die Erfindung mittels zweier Figuren näher erläutert. Dazu zeigen
- Fig.1: einen Querschnitt durch eine Kanüle mit einem Griff, bestehend aus einem Hauptteil und zwei Anschlussstücken,
- Fig.2: einen Längsschnitt durch einen von den beiden Anschlussstücken gebildeten Körper.

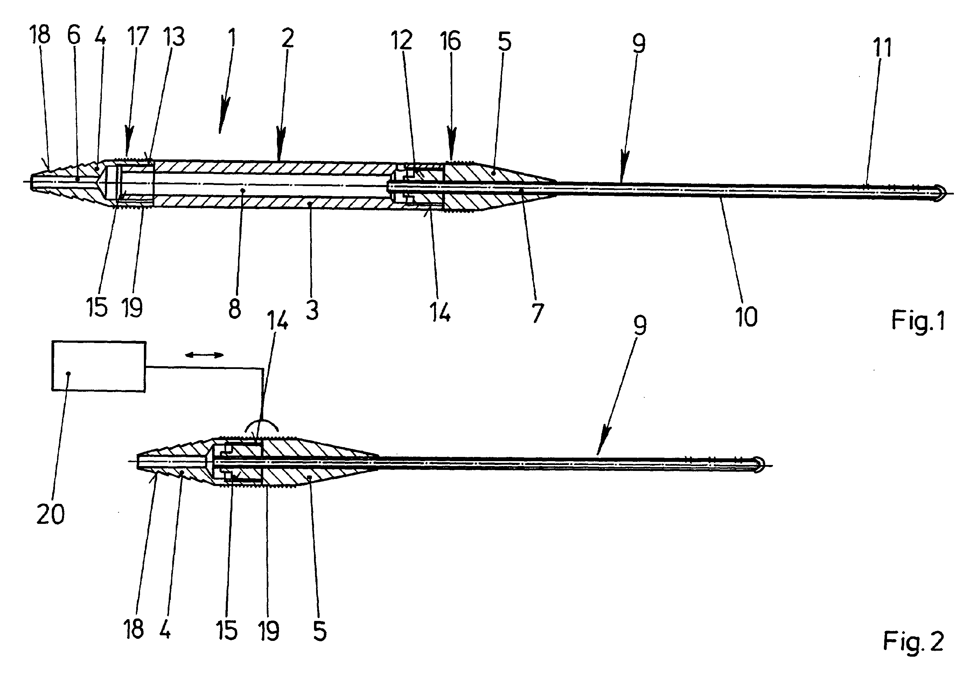

Zunächst wird auf die Figur 1 Bezug genommen. Ein Griff 1 besteht aus einem einen Hauptteil 2 des Griffes 1 bildenden Rohr 3 sowie einem hinteren Anschlussstück 4 und einem vorderen Anschlussstück 5. Diese sind jeweils mit Längskanälen 6, 7 versehen, die mit dem Rohrkanal 8 fluchten. Eine Kanüle 9 besteht aus einem Kanülenrohr 10, das wie üblich vorne geschlossen und seitlich mit Ansaugöffnungen 11 versehen ist. Das hintere Ende des Kanülenrohres 10 steckt im Längskanal 7 des vorderen Anschlussstückes 5 und ist dort verschweißt, verlötet oder eingeschrumpft. Das hintere Anschlussstück 4 wird mit einer hier nicht näher dargestellten Saugpumpe verbunden.

Das Rohr 3 ist an seinem vorderen Ende mit einem Innengewinde 12 und an seinem hinteren Ende mit einem Außengewinde 13 versehen. Dementsprechend weist das vordere Anschlussstück 5 ein Außengewinde 14 auf, auf das das vordere Innengewinde 12 des Rohres 3 aufschraubbar ist.

Das hintere Anschlussstück 4 weist ein Innengewinde 15 auf, mit dem es auf das hintere Außengewinde 13 des Rohres 3 aufschraubbar ist.

Jedes Anschlussstück 4, 5 weist einen an das Rohr 3 anschließenden Hohlzylinder 16, 17 auf, deren Durchmesser identisch ist mit dem Außendurchmesser des Rohres 3, so dass sich ein glatter Übergang ergibt. Im Bereich der Hohlzylinder 16, 17 sind die Anschlussstücke 4, 5 mit Riffelungen 19 versehen.

Das hintere Anschlussstück 4 läuft nach hinten konisch aus und ist mit einer tannenförmigen Struktur 18 versehen, auf der ein Schlauch aufsteckbar ist, mit dem der Griff 1 mit einer Saugpumpe verbunden ist.

Auch das vordere Anschlussstück 5 ist mit einem konischen, nach vorne gerichteten Ansatz versehen, so dass sich ein gleitender Übergang zum Kanülenrohr 10 ergibt.

Wie Figur 2 zeigt, entspricht der Durchmesser des Außengewindes 14 am vorderen Anschlussstück 5 dem Durchmesser des Innengewindes 15 des hinteren Anschlussstückes 4, so dass diese unmittelbar miteinander verschraubt werden können. Damit bildet sich ein Körper, der vom Greifer eines Vibrationsantriebssystems 20, das hier schematisch dargestellt ist, erfasst werden kann.

Ein Loch 21 im Rohrmantel ermöglicht die Steuerung der Saugleistung: Wenn der Operateur das Loch 21 mit seinem Finger schließt, wird die Saugleistung erhöht. Bleibt es offen, so wird über das Loch 21 Nebenluft angesaugt, was die Saugleistung herabsetzt.

### Bezugszeichenliste

- 1: Griff
- 2: Hauptteil
- 3: Rohr
- 4: Anschlussstück
- 5: Anschlussstück

- 6: Längskanal
- 7: Längskanal
- 8: Rohrkanal
- 9: Kanüle
- 10: Kanülenrohr

- 11: Ansaugöffnung
- 12: Innengewinde
- 13: Außengewinde
- 14: Außengewinde
- 15: Innengewinde

- 16: Hohlzylinder
- 17: Hohlzylinder
- 18: Tannenstruktur
- 19: Riffelung
- 20: Vibrationsantriebssystem

- 21: Loch

## Patentansprüche

1. Instrument zum Absaugen von Körperfett mit einer hohlen Kanüle (9) und einem einen Hauptteil (2) und zwei Anschlussstücke (4, 5) aufweisenden hohlen Griff (1), wobei an das eine Anschlussstück (4) eine Saugpumpe anschließbar und das andere Anschlussstück (5) mit der Kanüle (9) verbunden ist, **dadurch gekennzeichnet, dass** die beiden Anschlussstücke (4, 5) des Griffes (1) vom Hauptteil (2) des Griffes (1) lösbar und zu einem mit einem Vibrationsantriebssystem (20) koppelbaren Körper zusammenbringbar sind.

2. Instrument nach Anspruch 1, **dadurch gekennzeichnet, dass** die Anschlussstücke (4, 5) über gleichartig aufgebaute, jeweils aus zwei korrespondierenden Verbindungselementen bestehenden Verbindungen mit dem Hauptteil (2) koppelbar sind, wobei die Verbindungen bezüglich des Hauptteils (2) spiegelsymmetrisch sind, so dass die Verbindungselemente der beiden Anschlussstücke (4, 5) miteinander verbindbar sind, um den mit dem Vibrationsantriebssystem (20) koppelbaren Körper zu bilden.

3. Instrument nach Anspruch 2, **dadurch gekennzeichnet, dass** es sich bei den Verbindungen jeweils um Schraubverbindungen handelt, wobei das eine Verbindungselement ein Innengewinde (12, 15) und das andere ein dazu passendes Außengewinde (14, 13) ist, und dass am Hauptteil (2) des Griffes (1) ein Innengewinde (12) und ein Außengewinde (13) ausgebildet ist.

4. Instrument nach Anspruch 2 oder 3 **dadurch gekennzeichnet, dass** der Hauptteil (2) des Griffes (1) aus einem Rohr (3) besteht und die Anschlussstücke (4, 5) jeweils ein Hohlzylinder (16, 17), an dem ein Verbindungselement ausgebildet ist, beinhaltet, wobei der Außendurchmesser jedes Hohlzylinders (16, 17) dem des Rohres (3) entspricht.

5. Instrument nach Anspruch 4, **dadurch gekennzeichnet, dass** zumindest der Hohlzylinder (16) des Anschlussstückes (5), das mit der Kanüle verbunden ist, eine äußere Mantelfläche mit einer Riffelung (19) aufweist.

6. Instrument nach einem der vorhergehende Ansprüche, **dadurch gekennzeichnet, dass** die Kanüle (9) nicht lösbar mit ihrem Anschlussstück (5) verbunden ist.
